# EUROPEAN PATENT SPECIFICATION

(11) **EP 2 739 206 B1**
(45) Date of publication and mention of the grant of the patent: **17.05.2017**
(21) Application number: 12820383.3
(22) Date of filing: 18.07.2012
(51) Int. Cl.: A61B 5/00

(54) **ADAPTIVE ILLUMINATION METHOD AND APPARATUS FOR DENTAL SHADE MATCHING**
ADAPTIVES BELICHTUNGSVERFAHREN UND VORRICHTUNG ZUR ZAHNSCHATTIERUNGSANPASSUNG
PROCÉDÉ ET APPAREIL D'ÉCLAIRAGE ADAPTATIF PERMETTANT DE S'ASSORTIR AUX NUANCES DENTAIRES

(30) Priority: 02.08.2011 US 201113196137
(43) Date of publication of application: 11.06.2014
(73) Proprietor: Carestream Health, Inc., Rochester, NY 14608 (US)
(72) Inventor: LIANG, Rongguang, Rochester, NY 14608 (US); MILCH, James, Rochester, NY 14650 (US); INGLESE, Jean-marc, Rochester, NY 14608 (US)
(74) Representative: Emde, Eric
(86) International application number: PCT/US2012/047096
(87) International publication number: WO 2013/019405

(56) References cited:
- EP-A1- 1 150 159
- EP-A1- 2 258 254
- EP-A1- 2 258 254
- JP-A- 2010 194 296
- US-A1- 2005 123 180
- US-A1- 2005 244 794
- US-B1- 6 201 880
- US-B1- 6 206 691

## Description

### FIELD OF THE INVENTION

The invention generally relates to imaging of small objects and more particularly relates to an apparatus and method for shape and color shade imaging of the surface of teeth and other structures.

### BACKGROUND OF THE INVENTION

Modem restorative dental procedures often require accurate color matching, such as for filling materials and for the fabrication of restorations such as crowns, implants, fixed partial dentures, and veneers. The materials used for these procedures, such as ceramics and other materials, can be skillfully formed and treated to closely match the shape, texture, color and translucency of natural teeth.

One technique for determining and communicating tooth color information is a process referred to as "shade matching" whereby the dentist or technician visually matches a patient's tooth to one of a number of reference shade samples or shade tabs within one or more sets of standardized shade guides. The practitioner who performs the match records the identification of the matching shade tab and conveys that information to the dental laboratory where the restoration or prosthesis is then fabricated. The laboratory then uses its own set of the same shade guides to perform visual color evaluations of the restoration or prosthesis throughout the fabrication process.

The visual shade matching process can be highly subjective and subject to a number of problems. The initial matching procedure is often difficult and tedious, and it is not unusual for the process to take twenty minutes or longer. In many cases, there is no shade tab that perfectly matches the patient's teeth. Tooth color itself results from a relatively complex interaction of reflection, transmission, refraction, fluorescence, and scattering by a variety of organic and inorganic components. It is influenced by variations in tooth pulp volume, dentin condition, enamel composition, and other variations in the composition, structure, and thickness of the dental tissues. One result of this complexity is that color appearance and color measurement are greatly influenced by lighting geometry, spectrum, surrounding colors, and other environmental factors.

As a further complication, color within a single tooth is generally not uniform. Color non-uniformities can result from spatial variations in composition, structure, thickness, internal and external stains, surface texture, fissures, cracks, and degree of wetness. As a result, measurements taken over relatively large areas produce averaged values that may not be representative of a tooth's dominant color. In addition, natural color variations and non-uniformities make it unlikely that a given tooth can be matched exactly by any single shade tab. People are generally particular about the appearance of their teeth. Understandably, they are quite intolerant of restorations that appear inappropriate in color.

In cosmetic dentistry, the fabrication lab may require additional information in order to more accurately map tooth color in addition to simple shade matching. In practice, the dentist or technician may provide a photograph in addition to a shade tab, so that the fabrication lab can adjust color characteristics over different portions of the tooth. This helps to provide a type of color mapping for subjective use, with information that relates to the shade tab and shows how colors in other portions of the tooth vary from that of the shade tab.

Among problems inherent to dental imaging is the difficulty in providing sufficient light to the tooth of interest. Light-emitting diodes (LEDs) can be positioned near the tooth surface, such as the imaging camera described in U.S. Patent No. 5251025 entitled "Electronic Video Dental Camera" (Cooper). Fiber optic light might be employed, such as described in U.S. Patent No. 5027138 entitled "Dental Camera System" (Gandrud).

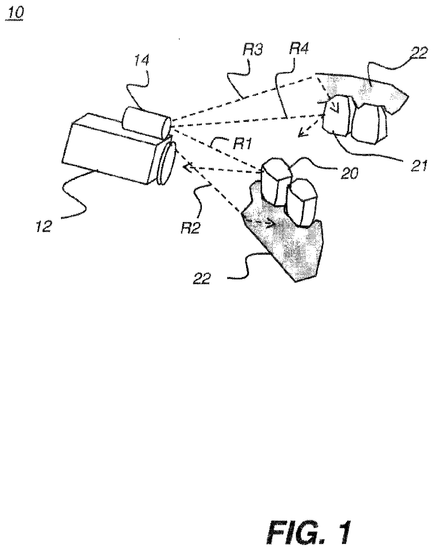

While cameras might illuminate the tooth in some applications, there are drawbacks. One issue relates to the light distribution pattern itself. Referring to Figure 1, there is shown a conventional intra-oral imaging apparatus 10 that has a camera 12 and a light source 14. To obtain an image of a tooth 20, light source 14 is energized to direct light toward tooth 20 and surrounding structures. Light along ray R1 illuminates the object of interest, tooth 20, and is detected at camera 12. The spectral content of light from R1 correctly shows the color of tooth 20 in the image. However, stray light introduces other spectral content that confounds the color data that has been obtained. Light along ray R2, for example, reflects from another tooth or other surface inside the mouth and can introduce some unwanted spectral content. Ray R3 reflects from a gum surface 22 or other tissue surface. Light reflecting from the gum tissue can find its way back to camera 12 and affect the color of the image data. Light along ray R4 reflects from another tooth 21, not the tooth of interest. Among issues with scattered light are variations in spectral content. Scattered light tends to contribute more in the red parts of the visible spectrum than in the blue, causing some unwanted color shift. It is not practical to mask the scattered light, preventing some portion of this light from reaching the tooth of interest. Still other issues with the conventional arrangement shown in Figure 1 relate to light from specular reflection from the tooth surface itself. Further, reference is made to EP 2 258 254, which discloses an intra-oral imaging apparatus having an illumination field generator that forms an illumination beam having a contour fringe projection pattern when receiving light from a first light source and having a substantially uniform illumination field when receiving light from a second light source. A polarizer in the path of the illumination beam has a first polarization transmission axis. A projection lens directs the polarized illumination beam toward a tooth surface and an imaging lens directs at least a portion of the light from the tooth surface along a detection path. A polarization-selective element disposed along the detection path has a second polarization transmission axis. At least one detector obtains image data from the light provided through the polarization-selective element. A control logic processor responds to programmed instructions for alternately energizing the first and second light sources in a sequence and obtaining both contour fringe projection data and color image data. US 2005 123 180 A1 relates to a method, computer program, and system for locating a tooth within a digital dental image. A reference object that was placed in the patient's mouth is segmented within a digital dental image to provide a segmented reference. The reference object has a predetermined size dimension. A window is segmented in the dental image at the position of the dental target. The segmented reference defines the relative size and location of the window, prior to the segmenting of the window.

Unwanted spectral content from light that scatters or reflects from surrounding structures is unavoidable with conventional designs and makes it difficult to perform accurate shade matching with existing intra-oral cameras. There is a need for a color matching apparatus and method that provide an image of a tooth or other object, wherein the image data that is obtained more accurately represents the spectral content of a tooth.

### SUMMARY OF THE INVENTION

It is an object of the present invention to advance the art of shade matching in intra-oral and other imaging applications. Embodiments of the present invention help to provide an image of a tooth or other feature with reduced spectral content from surrounding tissue or other features. Illumination that is directed toward the tooth or other feature is cross-sectionally shaped according to segmentation data that is obtained from an image of the tooth and nearby structures. Advantageously, stray illumination is reduced, allowing a more accurate measure of the color of the tooth or other feature of interest to be obtained.

These objects are given only by way of illustrative example, and such objects may be exemplary of one or more embodiments of the invention. Other desirable objectives and advantages inherently achieved by the disclosed invention may occur or become apparent to those skilled in the art. The invention is defined by the appended claims.

According to one aspect of the invention, there is provided a method as set forth in claim 1. Further embodiments are inter alia disclosed in the dependent claims. Such a method may inter performed on an intra-oral imaging apparatus for obtaining an image of a tooth, comprising: an image capture apparatus comprising an imaging sensor that is energizable to obtain image data and one or more optical elements for directing light from the tooth to the imaging sensor; an illumination apparatus comprising one or more light sources energizable to emit light and a spatial light modulator that is configurable to shape an illumination beam from the emitted light; one or more optical elements for relaying the shaped illumination beam toward the tooth surface; and a control logic processor in signal communication with the imaging sensor for obtaining image data and in signal communication with the spatial light modulator for shaping the illumination beam according to the obtained image data.

### BRIEF DESCRIPTION OF THE DRAWINGS

The foregoing and other objects, features, and advantages of the invention will be apparent from the following more particular description of the embodiments of the invention, as illustrated in the accompanying drawings. The elements of the drawings are not necessarily to scale relative to each other.
Figure 1 shows a perspective view of a conventional imaging apparatus for intra-oral imaging.
Figure 2 is a logic flow diagram showing a sequence for imaging a tooth for color shade matching according to an embodiment of the present invention.
Figure 3 shows a sequence of images and illumination masking used for shade color matching.
Figure 4 is a logic flow diagram showing a sequence for imaging multiple teeth according to an embodiment of the present invention.
Figures 5A and 5B contrast results from conventional illumination against results using the masked illumination according to embodiments of the present invention.
Figure 6 is a schematic block diagram that shows components of an imaging apparatus that uses the conditioned illumination of the present invention.
Figure 7 is a schematic block diagram that shows components of an imaging apparatus that uses the conditioned illumination of the present invention in an alternate embodiment of the present invention.

### DETAILED DESCRIPTION OF THE INVENTION

The following is a detailed description of the preferred embodiments of the invention, reference being made to the drawings in which the same reference numerals identify the same elements of structure in each of the several figures. Where they are used, the terms "first", "second", and so on, do not necessarily denote any ordinal or priority relation, but may be simply used to more clearly distinguish one element from another.

Figures provided herein are given in order to illustrate key principles of operation and component relationships along their respective optical paths according to the present invention and are not drawn with intent to show actual size or scale. Some exaggeration may be necessary in order to emphasize basic structural relationships or principles of operation. Some conventional components that would be needed for implementation of the described embodiments, such as support components used for providing power, for packaging, and for mounting and protecting system optics, for example, are not shown in the drawings in order to simplify description of the invention itself. In the drawings and text that follow, like components are designated with like reference numerals, and similar descriptions concerning components and arrangement or interaction of components already described are omitted.

Embodiments of the present invention address the problem of color shade identification. For this reason, unless otherwise specified, references to a tooth relate to visible surfaces of the tooth for which color match is a concern.

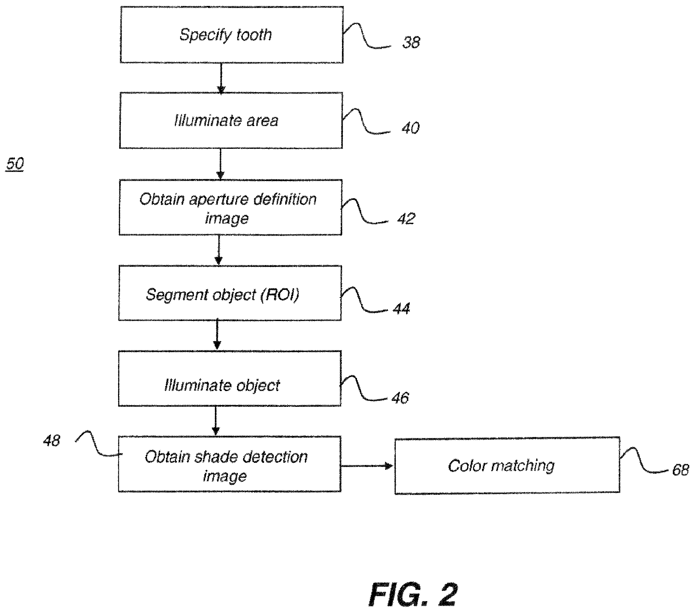

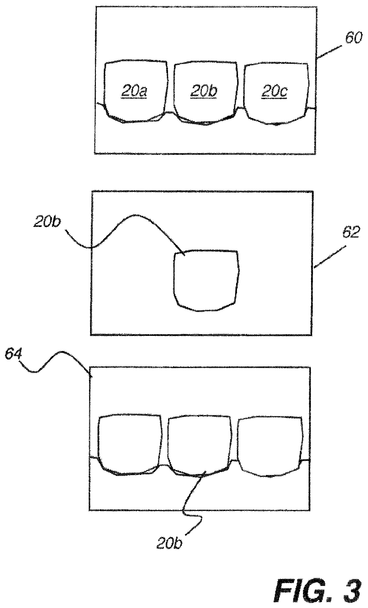

Embodiments of the present invention address the problem of stray light in dental imaging described previously with reference to Figure 1 and condition the illumination to direct light only to the surface of the tooth of interest. The logic flow diagram of Figure 2 shows an imaging sequence 50 of steps used to address this problem, consistent with an embodiment of the present invention. The plan view of Figure 3 then shows the sequence of image capture and image processing that is used for the process described in Figure 2.

In an optional specification step 38 in Figure 2, the technician or practitioner identifies a tooth 20b as the tooth of interest for which a color match is desired. In a broad area illumination step 40, tooth 20b and nearby teeth 20a and 20c and surrounding tissue are illuminated and an aperture definition image 60 is captured in an image capture step 42. As noted previously, due to stray light reflected and scattered from a number of surfaces, this image can be poorly suited for obtaining accurate information for a color match of tooth 20b, but is used instead for segmentation that defines the shape of an aperture in the illumination path. A segmentation step 44 segments tooth 20b within aperture definition image 60, defining an area within the image that then serves as a mask for conditioning the illumination for obtaining a second image. Tooth segmentation is known in the dental imaging arts, and any of a number of tooth segmentation algorithms can be used for this purpose. As an example of tooth segmentation processing, U.S. Patent Application Publication No. 2005/0244794 entitled "Computer-implemented system and method for automated and highly accurate plaque analysis, reporting, and visualization" (Kemp) describes variations on a watershed segmentation algorithm that is known to those skilled in the image processing arts.

Continuing with the sequence of Figure 2, segmentation data about tooth of interest 20b is then used to shape an aperture that provides a mask for conditioning the illumination beam. A variable aperture in the illumination path cross-sectionally shapes the beam so that it illuminates only the surface of the tooth of interest in a masked illumination step 46. This use of masked illumination is shown as masked illumination 62 in Figure 3. A second image capture step 48 then obtains a color shade detection image 64, with the cross-sectionally shaped illumination beam directed only toward the tooth of interest as shown in Figure 3. In one embodiment, color shade detection image 64 is obtained under polychromatic illumination, in one image capture operation. In an alternate embodiment, color shade detection image 64 is obtained as a composite of individual images taken in close sequence, such as a first image obtained using Red illumination, a second image obtained using Green illumination, and a third image obtained using Blue illumination, for example. A color matching step 68 then performs the color matching algorithms for obtaining a suitable color match for the tooth of interest and reports and stores results in an electronic memory.

Optional identification step 38 for identifying the tooth of interest can be executed in a number of ways. Consistent with one embodiment, a laser pointing beam is provided to identify the tooth of interest and to center this tooth in the image that is obtained in image capture step 42. In an alternate embodiment, aperture definition image 60 displays on a display monitor, allowing operator selection of a particular tooth within aperture definition image 60 as the tooth of interest. A touch screen or other operator interface is provided for identifying the tooth of interest.

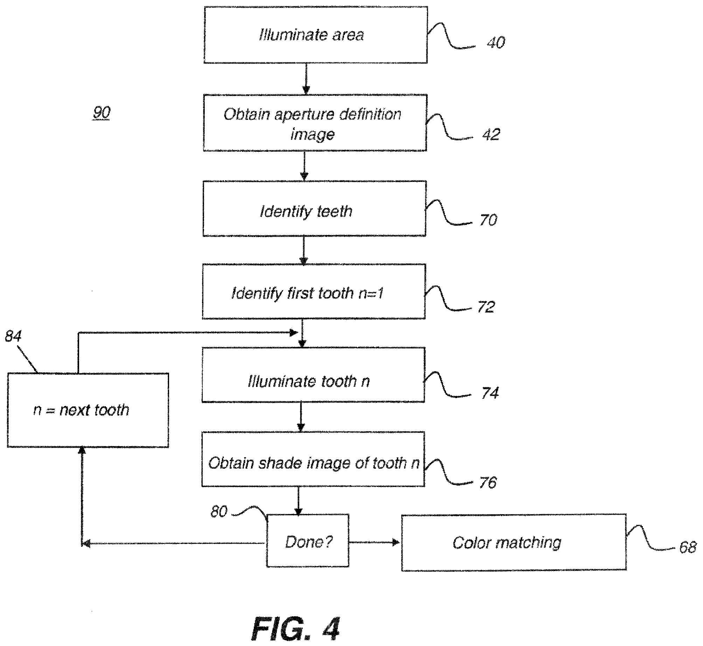

It is noted that specification step 38 of Figure 2 can be executed automatically, in part of a looping operation that successively identifies each tooth in aperture definition image 60. Referring to the logic flow diagram of Figure 4, there is shown an automated imaging sequence 90 that automates the segmentation, masked illumination, and imaging processes in an alternate embodiment, so that each tooth in aperture definition image 60 (Figure 3) is successively selected and imaged without further operator interaction. Illumination step 40 executes as described with reference to Figure 2, and is followed by first image capture step 42 that obtains an aperture definition image for segmentation analysis in an identification step 70. As part of step 70, multiple tooth surfaces are identified, such as using a process that performs segmentation within multiple portions of the image area. In a selection step 72, a first tooth surface is identified as tooth *n* = 1. An illumination step 74 shapes the aperture in the illumination path and illuminates tooth *n* using the shaped illumination beam. An image capture step 76 then captures a color shade detection image of tooth *n* for use in color shade analysis. A decision step 80 then determines if all teeth in identification step 70 have been imaged. If not, the process indexes to the next tooth, *n* = 2 in a next tooth selection step 84 and steps 74, 76, and 80 repeat for the next and any subsequent teeth until all of the tooth surfaces identified in identification step 70 have been imaged. Using this sequence, an imaging apparatus can be kept in the same position for obtaining color shade information from multiple teeth in one part of the patient's mouth.

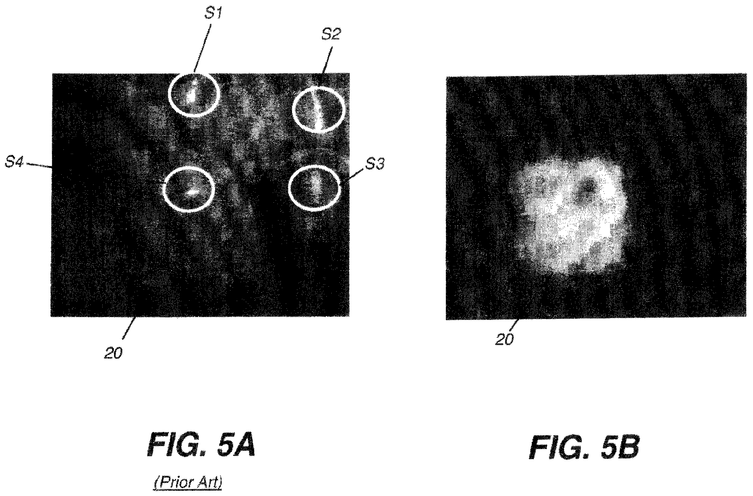

Images in Figures 5A and 5B contrast conventional tooth imaging results with results obtained using the imaging sequences described with reference to Figures 2, 3, and 4. Figure 5A shows an image of tooth 20 with unwanted specular reflection, as shown by circled areas S1, S2, S3, and S4. This reflected light can scatter and complicate color shade analysis for tooth 20, as described earlier with reference to Figure 1. Figure 5B, on the other hand, shows results using segmentation and masked illumination according to embodiments of the present invention. Tooth 20 is illuminated, and surrounding gums and nearby teeth are not illuminated, to obtain the image for color shade matching.

In addition to masking illumination from other teeth and other specular regions, embodiments of the present invention also detect and respond to highly specular reflection from areas on the tooth of interest. As shown as area S4 in Figure 5A, there is a region of tooth 20, area S4, that exhibits specular reflection. Embodiments of the present invention can handle this type of condition by masking some or all illumination from the area, yielding the results shown in Figure 5B. According to one embodiment of the present invention, light that is directed to this area is reduced, without reducing the light intensity to surrounding portions of tooth 20. In an alternate embodiment of the present invention, that portion of the image that shows specular reflection is simply not used in color shade calculation. It is then assumed that surrounding areas accurately provide color shade information for tooth 20.

### Imaging Apparatus

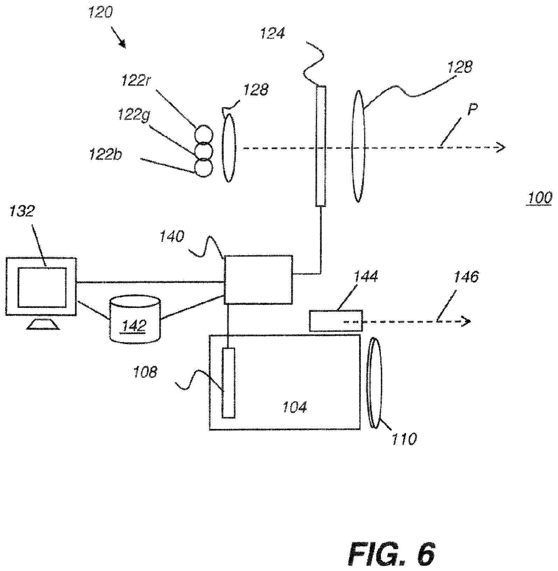

The schematic block diagram of Figure 6 shows an imaging apparatus 100 that is used to obtain aperture definition image 60 and to provide the masked illumination 62 and second image 64 of Figure 3 consistent with an embodiment of the present invention. An image capture apparatus 104 has an imaging sensor 108 that is energizable to obtain image data from a tooth or other object, along with suitable optics for directing light from the tooth, shown as a lens 110 but possibly including a number of refractive or reflective optical elements. An illumination apparatus 120 has one or more light sources 122r, 122g, and 122b, which may be Red, Green and Blue light emitting diodes (LEDs) or other sources that cooperate to emit, along an illumination path P, polychromatic light suitable for color matching. A single polychromatic light source, termed a white light source, can alternately be used. A spatial light modulator 124 is configurable as a variable aperture in the illumination path, to provide the light masking function of masked illumination 62 (Figure 3) that shapes an illumination beam for obtaining the second image 64. One or more lenses 128 are provided as optical elements for relaying the shaped illumination beam toward the surface of the tooth or other object of interest. A control logic processor 140, such as a dedicated microprocessor or other programmable logic device, is in signal communication with imaging sensor 108 and with spatial light modulator 124, as well as with light sources 122r, 122g, and 122b. Executing stored program instructions, control logic processor 140 obtains the aperture definition image 60, performs the needed segmentation sequence, and controls spatial light modulator 124 and light sources 122r, 122g, and 122b for obtaining first and second images according to the appropriate sequence described earlier with reference to Figures 2 through 4. An optional display 132 allows tooth selection and other operator control functions. An electronic memory 142, in signal communication with control logic processor 140, is used for storage of the obtained images.

Spatial light modulator 124 that provides an aperture in the illumination path can be any of a number of types of light conditioning element that is capable of forming a mask to shape the illumination beam. In one embodiment, spatial light modulator 124 is a liquid crystal diode (LCD) array that is energizable for selectively transmitting light according to a two-dimensional pattern. Alternate devices available include other types of light valves or light modulator arrays.

Figure 6 also shows an optional laser pointer 144 that is used as a light pointer to direct a laser beam 146 for pointing to the tooth or other object of interest for imaging. The location of laser beam 146 is then detected and used by control logic processor 140 to help isolate the tooth or other object of interest in the field of view for segmentation. For example, the tooth area pinpointed by the laser beam can be used to provide a seed for a segmentation algorithm.

Consistent with an embodiment of the present invention, the function of control logic processor 140 is executed by a dedicated microprocessor that is part of imaging apparatus 100. In an alternate embodiment, control logic processor 140 functions are executed, at least in part, on an external computer, such as on a networked computer or on some other logic processing apparatus.

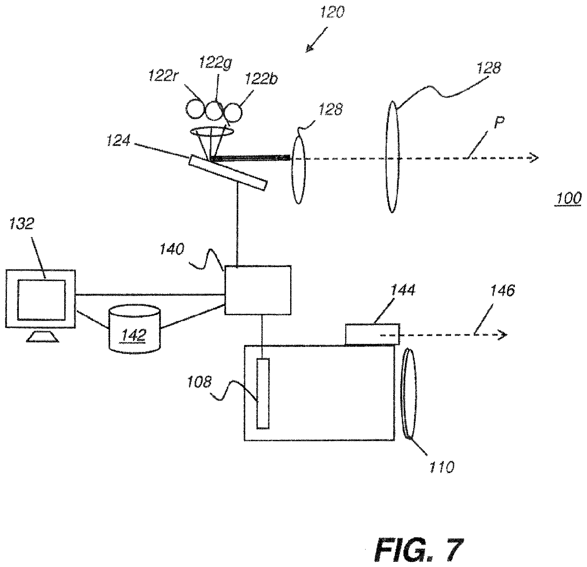

The schematic block diagram of Figure 7 shows imaging apparatus 100 in an alternate embodiment, in which spatial light modulator 124 is a digital micromirror device or other multi-element reflector array. Control logic processor 140 continues to be in signal communication with imaging sensor 108 for obtaining image data and with reflective spatial light modulator 124 serving as a variable aperture for beam-shaping along the illumination beam path P according to the image that is obtained.

The light that is provided for obtaining the second image that is used for color shade detection is polychromatic light, such as light from red, green, and blue LEDs or light from a single white light source. When multiple light sources are used, segmentation can be performed using an image obtained using monochromatic light, such as by energizing only the green LED in step 40 of Figure 2 to obtain the aperture definition image for segmentation, for example.

Imaging sensor 108 can be any of a number of types of imaging array, such as a complementary metal-oxide semiconductor (CMOS) array or a charge-coupled device (CCD).

By shaping the illumination beam in a cross-sectional manner, embodiments of the present invention direct light only to surfaces of the tooth of interest, reducing the amount of stray light that can reflect from nearby teeth and tissue structures and can adversely impact color shade matching.

The invention has been described in detail with particular reference to a presently preferred embodiment, but it will be understood that variations and modifications can be effected within the scope of the invention. While embodiments described herein are directed to intra-oral imaging, the methods of the present invention can alternately be used for imaging other small objects where obtaining a color match or other detailed data on the objects is of value.

Unless specifically stated otherwise as apparent from the preceding discussion, it is appreciated that throughout the description, discussions utilizing terms such as "processing" or "computing" or "calculating" or "determining" or "displaying" or "determining" or the like, refer to the action and processes of a computer system, or similar electronic computing device, that manipulates and transforms data represented as physical (electronic) quantities within the computer system memories or registers or other such information storage, transmission or display devices.

Certain aspects of the present invention include process steps and instructions described herein in the form of algorithms or image processing utilities. It should be noted that the process steps and instructions of the present invention could be embodied in software, firmware or hardware, and when embodied in software, could be downloaded to reside on and be operated from different platforms used by a variety of operating systems.

It should be noted that the term "memory", in the context of the present disclosure, can refer to any type of temporary or more enduring data storage workspace used for storing and operating upon image data in a system using a computer or other logic processor. The memory could be, for example, a long-term storage medium such as magnetic or optical storage. Alternately, the memory could be an electronic circuit, such as random-access memory (RAM) that is used as a temporary buffer or workspace by a microprocessor or other control logic processor device. Display data, for example, is typically stored in a temporary buffer and refreshed as needed in order to provide displayed data. This temporary storage buffer can also be considered to be a memory. Memory can be volatile, non-volatile, or a hybrid combination of volatile and non-volatile types.

## Claims

1. A method for obtaining a color shade of a tooth, the method comprising:
illuminating (40) a plurality of teeth (20 a, 20b, 20c) with a first illumination beam and obtaining (42) an aperture definition image (60) of the plurality of teeth (20 a, 20b, 20c);
segmenting (44) at least one tooth from within the aperture definition image (60) to obtain at least one segmented tooth (20b);
generating a second illumination beam based on the segmentation data of one of the at least one segmented tooth (20b) and cross-sectionally shaping the second illumination beam (46) in conformance with the one segmented tooth (20b) to generate a shaped illumination beam and illuminating only the one segmented tooth (20b) with the shaped illumination beam;
obtaining (48) a color shade detection image (64) of the one segmented tooth (20b) under illumination with the shaped illumination beam; and determining a color shade of the one segmented tooth (20b) from the color shade detection image (64).

2. The method of claim 1 wherein obtaining the aperture definition image (60) is performed using monochrome light and wherein obtaining the second color shade detection image (64) is performed using polychromatic light.

3. The method of claim 1 or 2 wherein cross-sectionally shaping the illumination beam (46) comprises directing the illumination beam toward a spatial light modulator.

4. The method of any one of claims 1 to 3, further comprising:
segmenting (70, 80, 84) a second tooth of the plurality of teeth from within the aperture definition image (60) to obtain a segmented second tooth;
generating a third illumination beam and cross-sectionally shaping the third illumination beam (74) in conformance with the segmented second tooth and illuminating only the segmented second tooth;
obtaining (76) another color shade detection image that comprises the segmented second tooth; and
determining the color shade (68) of the segmented second tooth from the another color shade detection image.

5. The method of claim 1 wherein obtaining the aperture definition image comprises illuminating the plurality of teeth with polychromatic light.

6. The method of claim 1 wherein one or more illumination sources of different respective wavelengths are used to generate a selected illumination beam.

7. The method of any one of claims 1 to 6, wherein cross-sectionally shaping the illumination beam (46) comprises shaping an aperture to form a mask allowing to illuminate only the surface of the segmented tooth.

## Patentansprüche

1. Verfahren zum Erfassen eines Farbtons von einem Zahn, wobei das Verfahren Folgendes aufweist:
Beleuchten (40) einer Vielzahl von Zähnen (20a, 20b, 20c) mit einem ersten Beleuchtungsstrahl und Erhalt (42) eines Blendendefinitionsbilds (60) der Vielzahl von Zähnen (20a, 20b, 20c);
Segmentieren (44) wenigstens eines Zahnes innerhalb des Blendendefinitionsbilds (60) zum Erfassen von wenigstens einem segmentieren Zahn (20b);
Erzeugen eines zweiten Beleuchtungsstrahls basierend auf den Segmentationsdaten von einem des wenigstens einen segmentierten Zahnes (20b) und Querschnittsformen des zweiten Beleuchtungsstrahls (46) in Übereinstimmung mit dem einen segmentierten Zahn (20b) zum Erzeugen eines geformten Beleuchtungsstrahls und zum Beleuchten nur des einen segmentierten Zahns (20b) mit dem geformten Beleuchtungsstrahl;
Erfassen (48) eines Farbtondetektionsbilds (64) des einen segmentierten Zahns (20b) unter Beleuchtung mit dem geformten Beleuchtungsstrahl; und
Bestimmen eines Farbtons des einen segmentierten Zahns (20b) von dem Farbtondetektionsbild (64).

2. Verfahren nach Anspruch 1, wobei das Erfassen des Blendendefinitionsbilds (60) erreicht wird durch Verwenden monochromen Lichts und wobei das Erfassen des zweiten Farbtondetektionsbilds (64) erreicht wird durch Verwenden polychromatischen Lichts.

3. Verfahren nach Anspruch 1 oder 2, wobei das Querschnittsformen des Beleuchtungsstrahls (46) das Leiten des Beleuchtungsstrahls in Richtung eines dreidimensionalen Lichtmodulators aufweist.

4. Verfahren nach einem der Ansprüche 1 bis 3, das ferner Folgendes aufweist:
Segmentieren (70, 80, 84) eines zweiten Zahns aus der Vielzahl von Zähnen innerhalb des Blendendefinitionsbilds (60) zum Erfassen eines segmentierten zweiten Zahns;
Erzeugen eines dritten Beleuchtungsstrahls und Querschnittsformen des dritten Beleuchtungsstrahls (74) in Übereinstimmung mit dem segmentierten zweiten Zahn und Beleuchten nur des segmentieren zweiten Zahns;
Erfassen (76) eines weiteren Farbtondetektionsbilds, das den segmentierten zweiten Zahn aufweist; und
Bestimmen des Farbtons (68) des segmentierten zweiten Zahns von dem weiteren Farbtondetektionsbild.

5. Verfahren nach Anspruch 1, wobei das Erfassen des Blendendefinitionsbilds das Beleuchten der Vielzahl von Zähnen mit polychromatischem Licht aufweist.

6. Verfahren nach Anspruch 1, wobei zum Erzeugen eines ausgewählten Beleuchtungsstrahls eine oder mehr Beleuchtungsquellen von verschiedenen einzelnen Wellenlängen verwendet werden.

7. Verfahren nach einem der Ansprüche 1 bis 6, wobei das Querschnittsformen des Beleuchtungsstrahls (46) das Formen einer Begrenzung zum Bilden einer Abdeckung, die es erlaubt nur die Oberfläche des segmentierten Zahns zu beleuchten, aufweist.

## Revendications

1. Procédé pour obtenir une teinte de couleur d'une dent, le procédé comprenant :
éclairer (40) une pluralité de dents (20a, 20b, 20c) à l'aide d'un premier faisceau d'éclairage et obtenir (42) une image de définition d'ouverture (60) de la pluralité de dents (20a, 20b, 20c) ;
segmenter (44) au moins une dent dans l'image de définition d'ouverture (60) pour obtenir au moins une dent segmentée (20b) ;
générer un deuxième faisceau d'éclairage sur la base des données de segmentation de l'une desdites au moins une dent segmentée (20b) et mettre en forme en coupe le deuxième faisceau d'éclairage (46) en conformité avec ladite dent segmentée (20b) pour générer un faisceau d'éclairage mis en forme et éclairer seulement ladite dent segmentée (20b) avec le faisceau d'éclairage mis en forme ;
obtenir (48) une image de détection de teinte de couleur (64) de ladite dent segmentée (20b) sous l'éclairage du faisceau d'éclairage mis en forme ; et
déterminer une teinte de couleur de ladite dent segmentée (20b) à partir de l'image de détection de teinte de couleur (64).

2. Procédé selon la revendication 1, dans lequel l'obtention de l'image de définition d'ouverture (60) est réalisée en utilisant de la lumière monochromatique et dans lequel l'obtention de la deuxième image de détection de teinte de couleur (64) est réalisée en utilisant de la lumière polychromatique.

3. Procédé selon la revendication 1 ou 2, dans lequel la mise en forme en coupe du faisceau d'éclairage (46) comprend de diriger le faisceau d'éclairage en direction d'un modulateur de lumière spatial.

4. Procédé selon l'une quelconque des revendications 1 à 3, comprenant en outre :
segmenter (70, 80, 84) une deuxième dent de la pluralité de dents à partir de l'intérieur de l'image de définition d'ouverture (60) pour obtenir une deuxième dent segmentée ;
générer un troisième faisceau d'éclairage et mettre en forme en coupe le troisième faisceau d'éclairage (74) en conformité avec la deuxième dent segmentée et éclairer seulement la deuxième dent segmentée ;
obtenir (76) une autre image de détection de teinte de couleur qui comprend la deuxième dent segmentée ; et
déterminer la teinte de couleur (68) de la deuxième dent segmentée à partir de l'autre image de détection de teinte de couleur.

5. Procédé selon la revendication 1, dans lequel l'obtention de l'image de définition d'ouverture comprend l'éclairage de la pluralité de dents avec de la lumière polychromatique.

6. Procédé selon la revendication 1, dans lequel une ou plusieurs sources d'éclairage ayant différentes longueurs d'onde respectives sont utilisées pour générer un faisceau d'éclairage sélectionné.

7. Procédé selon l'une quelconque des revendications 1 à 6, dans lequel la mise en forme en coupe du faisceau d'éclairage (46) comprend la mise en forme d'une ouverture pour former un masque permettant d'éclairer seulement la surface de la dent segmentée.
